# FASCICULE DE BREVET EUROPEEN

(11) **EP 1 367 061 B1**
(45) Date de publication et mention de la délivrance du brevet: **04.01.2006**
(21) Numéro de dépôt: 03291601.7
(22) Date de dépôt: 30.06.2003
(51) Int. Cl.: C07K 5/02, C07K 5/06, C07D 209/42

(54) **Nouveau procédé de synthèse du perindopril et de ses sels pharmaceutiquement acceptables**
Verfahren für die Synthese von Perindopril und seiner pharmazeutischen annehmbaren Salzen
Method for synthesis of perindopril and its pharmaceutically acceptable salts

(43) Date de publication de la demande: 03.12.2003
(73) Titulaire: Les Laboratoires Servier, 92415 Courbevoie Cedex (FR)
(72) Inventeur: Dubuffet, Thierry, 76210 Bolbec (FR); Lecouve, Jean-Pierre, 76600 Le Havre (FR)

(56) Documents cités:
- EP-A- 1 321 471
- WO-A-96/33984

## Description

La présente invention concerne un procédé de synthèse industrielle du perindopril de formule (I) : 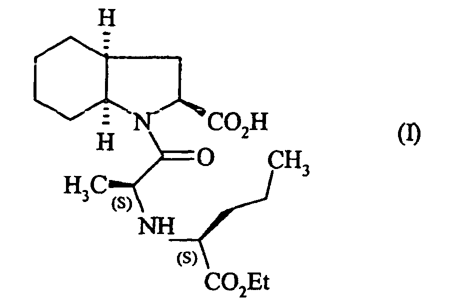 et de ses sels pharmaceutiquement acceptables.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir y accéder avec un procédé de synthèse industrielle performant, facilement transposable à l'échelle industrielle, conduisant au perindopril avec un bon rendement et une excellente pureté, à partir de matières premières bon marché.
Le brevet EP 0 308 341 décrit la synthèse industrielle du perindopril par couplage de type peptidique de l'ester benzylique de l'acide (2*S*, 3a*S*, 7a*S*)-octahydroindole 2-carboxylique avec l'ester éthylique de la N-[(S)-1-carboxybutyl]-(S)-alanine, suivie de la déprotection du groupement carboxylique de l'hétérocycle par hydrogénation catalytique.

Ce procédé présente l'avantage de conduire au perindopril avec un bon rendement, à partir de matières premières dont la synthèse industrielle est décrite.

Il présente cependant aussi des inconvénients liés à l'utilisation du dicyclohexylcarbodiimide dans l'étape de couplage : formation d'impuretés de couplage, ainsi que de dicyclohexylurée, un sous-produit difficile à éliminer.

La Demanderesse a présentement mis au point un nouveau procédé de synthèse industrielle du perindopril, qui évite la formation de ces produits secondaires.

Plus spécifiquement, la présente invention concerne un procédé de synthèse industrielle du perindopril, et de ses sels pharmaceutiquement acceptables, caractérisé en ce que l'on met en réaction le composé de formule (II) : 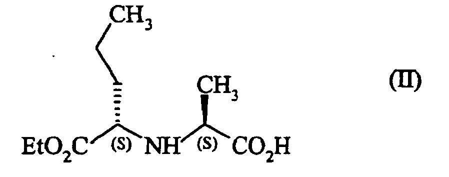 avec un composé de formule (III) : 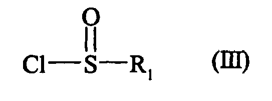
dans laquelle R₁ représente un groupement imidazolyle, benzimidazolyle ou tétrazolyle, pour conduire au composé de formule (IV) : 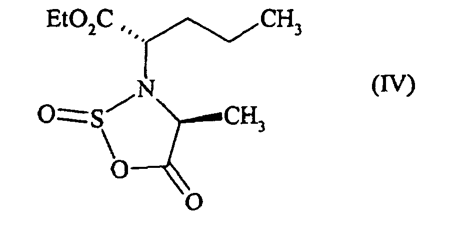 que l'on met en réaction avec un composé de formule (V) : 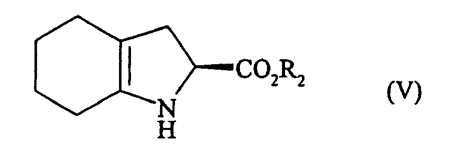
dans laquelle R₂ représente un atome d'hydrogène, un groupement benzyle ou alkyle (C₁-C₆) linéaire ou ramifié,
ou son sel d'addition à un acide minéral ou organique,
pour conduire après isolement au composé de formule (VI) : 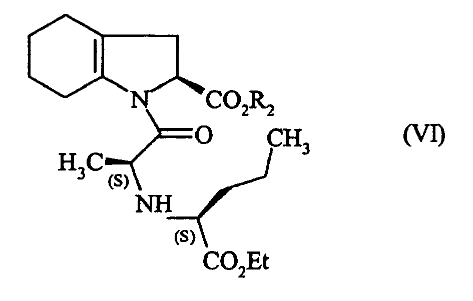
dans laquelle R₂ est tel que défini précédemment,
que l'on hydrogène en présence d'un catalyseur tel que, par exemple, le palladium, le platine, le rhodium ou le nickel,
sous une pression d'hydrogène comprise entre 1 et 30 bars, de préférence entre 1 et 10 bars, pour conduire, après déprotection de la fonction acide le cas échéant, au perindopril de formule (I), que l'on transforme, si on le souhaite, en un sel pharmaceutiquement acceptable tel que le sel de tert-butylamine.

L'exemple ci-dessous illustre l'invention, mais ne la limite en aucune façon.

### EXEMPLE : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

### Stade A : (2S)-2-[(4S)-4-Méthyl-2-oxydo-5-oxo-1,2,3-oxathiazolidin-3-yl]-pentanoate d'éthyle

Dans un réacteur, placer 200 g de N-[(S)-carbéthoxy-1-butyl]-(S)-alanine et 1,5 1 de dichlorométhane, puis ajouter à 0°C 325 g de chlorure de 1*H*-imidazole-1-sulfinyle.
Ramener ensuite le milieu réactionnel à température ambiante, puis, après 1 heure d'agitation, filtrer le précipité formé. Le filtrat obtenu est évaporé à sec, pour conduire au produit attendu sous la forme d'une huile.

### Stade B : Acide (2S)-1-{(2S)-2-[(1S)-1-(éthoxycarbonyl)-butylamino]-propionyl}--2,3,4,5,6, 7-hexahydro-1H-indole-2-carboxylique

Dans un réacteur, placer 200 g d'acide (2S)-2,3,4,5,6,7-hexahydro-1H-indole-2-carboxylique et 1,5 1 de dichlorométhane, puis 180 ml de triéthylamine.
Ajouter ensuite lentement une solution de 315 g du composé obtenu au stade précédent dans 500 ml de dichlorométhane, puis agiter 1 heure supplémentaire à température ambiante.
Après addition d'eau, le mélange réactionnel est ensuite refroidi à 15°C et le pH est amené à 4,2 par addition d'une solution d'acide chlorhydrique 2N. Après extraction, les phases organiques sont lavées puis évaporées pour conduire au produit attendu.

### Stade C : Acide (2S, 3aS, 7aS)-1-{(2S)-2-[(1S)-1-(éthoxycarbonyl)-butylamino]-propionyl}-octahydro-1H-indole-2-carboxylique

Dans un hydrogénateur, placer 200 g du composé obtenu dans le stade précédent, en solution dans l'acide acétique, puis 5 g de Pt/C à 10 %. Hydrogéner sous pression de 5 bars à température ambiante, jusqu'à absorption de la quantité théorique d'hydrogène.

Eliminer le catalyseur par filtration, puis refroidir entre 0 et 5°C et récolter le solide obtenu par filtration. Laver le gâteau et le sécher jusqu'à poids constant.

### Stade D : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

Le lyophilisat obtenu dans le stade précédent (200 g) est mis en solution dans 2,8 1 d'acétate d'éthyle, puis 40 g de tert-butylamine et 0,41 d'acétate d'éthyle sont ajoutés.
La suspension obtenue est ensuite portée au reflux jusqu'à dissolution totale, puis la solution obtenue est filtrée à chaud et refroidie sous agitation jusqu'à une température de 15-20°C.
Le précipité obtenu est alors filtré, réempâté à l'acétate d'éthyle, séché puis broyé pour conduire au produit attendu avec un rendement de 95 %.

## Revendications

1. Procédé de synthèse industrielle des composés de formule (I) : 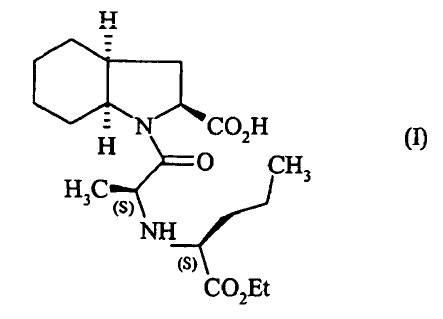 et de ses sels pharmaceutiquement acceptables,
**caractérisé en ce que** l'on met en réaction le composé de formule (II) : 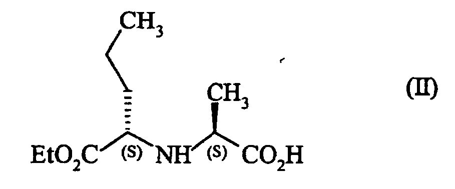
avec un composé de formule (III) : 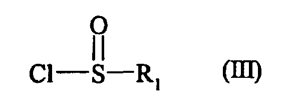
dans laquelle R₁ représente un groupement imidazolyle, benzimidazolyle ou tétrazolyle, pour conduire au composé de formule (IV) : 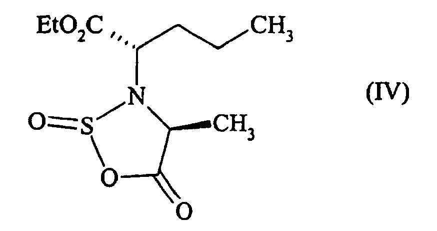
que l'on met en réaction avec un composé de formule (V) : 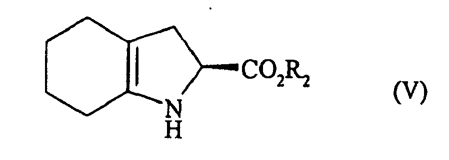
dans laquelle R₂ représente un atome d'hydrogène, un groupement benzyle ou alkyle (C₁-C₆) linéaire ou ramifié,
ou son sel d'addition à un acide minéral ou organique,
pour conduire après isolement au composé de formule (VI) : 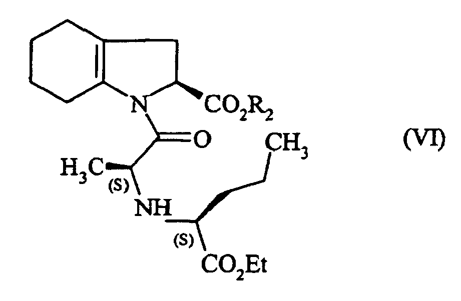
dans laquelle R₂ est tel que défini précédemment,
que l'on hydrogène en présence d'un catalyseur tel que, par exemple, le palladium, le platine, le rhodium ou le nickel,
sous une pression d'hydrogène comprise entre 1 et 30 bars, pour conduire, après déprotection de la fonction acide le cas échéant, au perindopril de formule (I), que l'on transforme, si on le souhaite, en un sel pharmaceutiquement acceptable tel que le sel de tert-butylamine.

2. Procédé de synthèse selon la revendication 1, **caractérisé en ce que** la pression d'hydrogène de la réaction d'hydrogénation est comprise entre 1 et 10 bars.

3. Procédé de synthèse selon la revendication 1 du perindopril sous sa forme de sel de tert-butylamine.

## Patentansprüche

1. Verfahren zur technischen Synthese der Verbindungen der Formel (I): 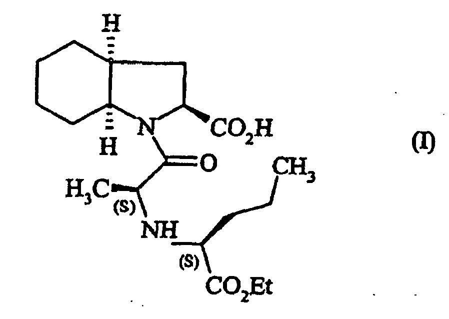
und von ihren pharmazeutische annehmbaren Salzen,
**dadurch gekennzeichnet, daß** man die Verbindung der Formel (II): 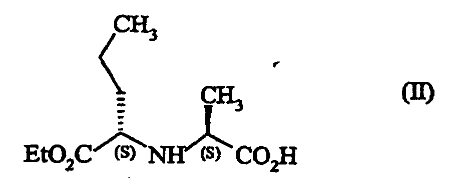
mit einer Verbindung der Formel (III): 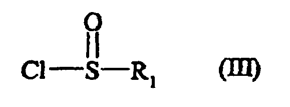
in der R₁ eine Imidazolyl-, Benzimidazolyl- oder Tetrazolylgruppe bedeutet, umsetzt,
zur Bildung der Verbindung der Formel (IV): 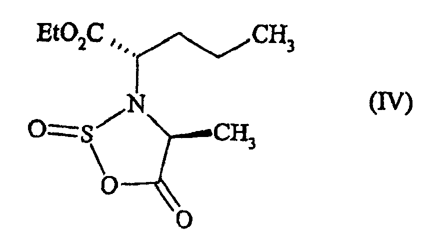
welche man mit einer Verbindung der Formel (V): 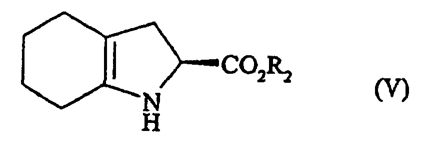
in der R₂ ein Wasserstoffatom, eine Benzylgruppe oder eine geradkettige oder verzweigte (C₁-C₆)-Alkylgruppe bedeutet,
oder seinem Additionssalz mit einer anorganischen oder organischen Säure umsetzt,
so daß man nach der Isolierung die Verbindung der Formel (VI) erhält: 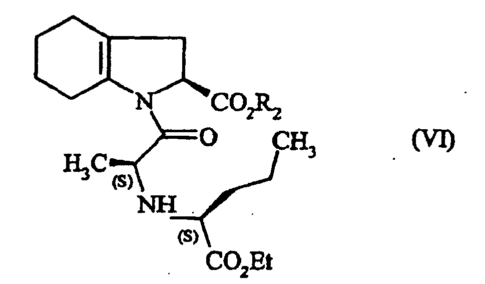
in der R₂ die oben angegebenen Bedeutungen besitzt,
welche man in Gegenwart eines Katalysators, wie beispielsweise Palladium, Platin, Rhodium oder Nickel,
bei einem Wasserstoffdruck zwischen 1 und 30 bar hydriert, so daß man gegebenenfalls nach der Abspaltung der Schutzgruppe der Säurefunktion Perindopril der Formel (I) erhält, welches man gewünschtenfalls in ein pharmazeutisch annehmbares Salz, wie das tert.-Butylaminsalz, umwandelt.

2. Syntheseverfahren nach Anspruch 1, **dadurch gekennzeichnet, daß** der Wasserstoffdruck der Hydrierungsreaktion zwischen 1 und 10 bar liegt.

3. Verfahren nach Anspruch 1 zur Synthese von Perindopril in Form seines tert.-Butylaminsalzes.

## Claims

1. Process for the industrial synthesis of the compounds of formula (I) : 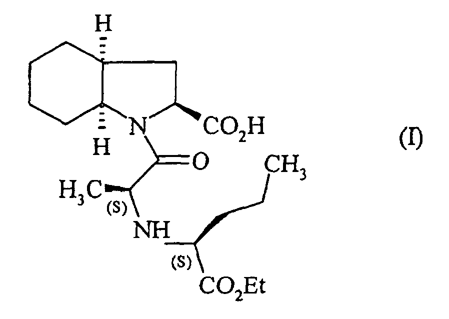
and its pharmaceutically acceptable salts,
**characterised in that** the compound of formula (II) : 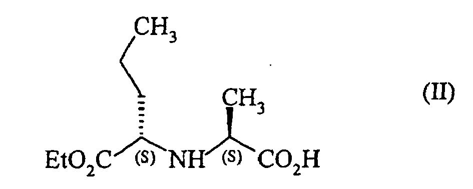
is reacted with a compound of formula (III) : 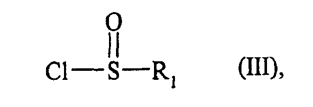
wherein R₁ represents an imidazolyl, benzimidazolyl or tetrazolyl group, to yield the compound of formula (IV) : 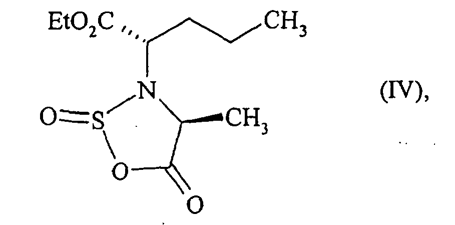
which is reacted with a compound of formula (V) : 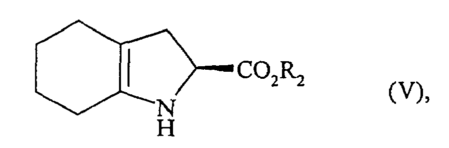
wherein R₂ represents a hydrogen atom, or a benzyl or linear or branched (C₁-C₆)alkyl group,
or an addition salt thereof with a mineral or organic acid,
to yield, after isolation, a compound of formula (VI) : 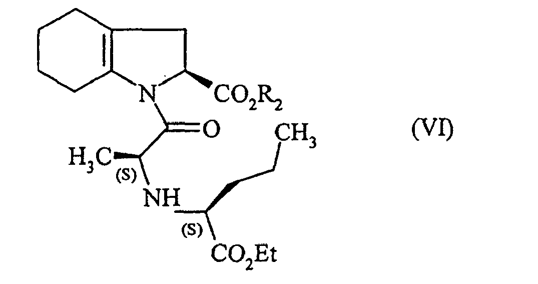
wherein R₂ is as defined hereinbefore,
which is hydrogenated in the presence of a catalyst such as, for example, palladium, platinum, rhodium or nickel,
under a hydrogen pressure of from 1 to 30 bars, to yield, after deprotection of the acid function where necessary, perindopril of formula (I), which is converted, if desired, into a pharmaceutically acceptable salt, such as the tert-butylamine salt.

2. Synthesis process according to claim 1, **characterised in that** the hydrogen pressure in the hydrogenation reaction is from I to 10 bars.

3. Process according to claim 1 for the synthesis of perindopril in the form of its tert-butylamine salt.
